# EUROPEAN PATENT SPECIFICATION

(11) **EP 1 799 229 B1**
(45) Date of publication and mention of the grant of the patent: **31.12.2008**
(21) Application number: 05796923.0
(22) Date of filing: 03.10.2005
(51) Int. Cl.: A61K 31/70, A61K 38/14, A61K 9/12, A61P 9/10

(54) **PULMONARY ADMINISTRATION OF AN ANTITHROMBOTIC COMPOUND**
PULMONÄRE VERABREICHUNG EINER ANTITHROMBOTISCHEN VERBINDUNG
ADMINISTRATION PULMONAIRE D'UN COMPOSE ANTITHROMBOTIQUE

(30) Priority: 06.10.2004 EP 04104897
(43) Date of publication of application: 27.06.2007
(73) Proprietor: N.V. Organon, 5349 AB Oss (NL)
(72) Inventor: VOGEL, Gerardus, Martinus, Theodorus, NL-5340 BH OSS (NL)
(74) Representative: van Wezenbeek, Petrus M.G.F.
(86) International application number: PCT/EP2005/054973
(87) International publication number: WO 2006/037771

(56) References cited:
- WO-A-01/42262
- WO-A-99/65934
- VOGEL G M T, MEULEMAN D G, VAN DINTHER T G, BUIJSMAN R, PRINCEN A W M, SMIT M J: "Antithrombotic properties of a direct thrombin inhibitor with a prolonged half life and AT-mediated factor Xa inhibitory activity" JOURNAL OF THROMBOSIS AND HAEMOSTASIS, vol. 1, no. 9, September 2003 (2003-09), pages 1945-1954, XP008045748 cited in the application

## Description

The invention relates to the use of oligosaccharides conjugated to a direct thrombin inhibitor for the manufacture of a medicament for the pulmonary delivery of said conjugates for the treatment or prevention of thrombosis or related disorders.

Recently, the search for new compounds for the treatment and prevention of thrombosis and related disorders has resulted in the invention of new compounds which are oligosaccharides conjugated to a direct thrombin inhibitor. The new compounds are dual inhibitors having direct anti-thrombin (factor IIa) activity and anti-thrombin III (AT-III) mediated anti-Xa activity (WO99/65934 Bioorg. Med. Chem. Letters 1999; 9: 13-18).

Improvements of those compounds followed, the new dual inhibitors having a pharmacological profile with less side-effects and a half-life allowing once-a-day treatment (WO01/42262; J. Thromb. Haem. 2003; 1: 1945-1954).

Although the new antithrombotic compounds are clinically highly interesting compounds, they suffer from the drawback that - until now - the only effective route of administration is by injection (*subcutaneous -* s.c. - or *intra venous -* i.v.). As a result, the clinical use of these compounds is essentially limited to hospital settings or other situations where phycisians or other health care professionals are involved. Consequently, the compounds will find their use mostly in acute settings. In addition to the inconvenience of s.c. or i.v. injection, injection is further associated with side effects such as local irritation and ulceration. Chronic anticoagulant therapy is therefore practically not an option with these antitrombotic compounds.

Therefore, there is a need for a convenient noninvasive alternative route of administration for said antithrombotic compounds with a performance comparable to parenteral injection.

Heparin and low-molecular-weight heparins (LMWH) suffer from similar disadvantages with respect to the route of administration as described above for the new oligosaccharide conjugate compounds. Recently, it was reported that aerosol solid particle formation of heparins and LMWHs can result in the efficient pulmonary delivery of that type of molecules (WO 02/32406; PNAS June 29,2004; Vo1.101, No.26, 9867-9872). It was discovered that pulmonary delivery depends on optimal formulation of dry aerosol particles. Absolute bioavailability of heparin, depending on formulation, was measured as 35 - 60 % by the method described. A significant difference in the pulmonary delivery of heparin was observed between the inhalation of dry aerosol particles and the instillation of heparin in liquid form into the lungs. Instillation of liquid heparin into the lungs of rabbits resulted in low bioavailability and poor pharmacokinetics.

Surprisingly, in contrast to the finding that instillation of liquid heparin into the lungs is essentially ineffective, it has now been found that pharmaceutically acceptable solutions of the oligosaccharide compounds of WO99/65934 and WO O1/42262 may be used for the pulmonary delivery of said compounds. Unexpectedly high bioavailibility (>70 %) was measured.

Therefore, the present invention relates to the use of a compound of the formula (I) 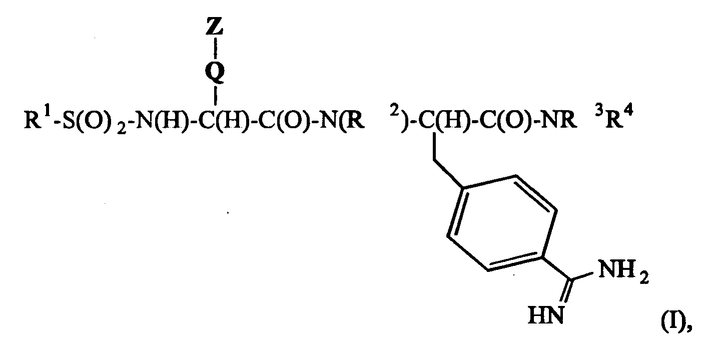
wherein R¹ is phenyl, naphthyl, 1,2,3,4-tetrahydronaphthyl, (iso)quinolinyl, tetrahydro(iso)quinolinyl, 3,4-dihydro-1H-isoquinolinyl, chromanyl or the camphor group, which groups may optionally be substituted with one or more substituents selected from (1-8C)alkyl or (1-8C)alkoxy;
R² and R³ are independently H or (1-8C)alkyl;
R⁴ is (1-8C)alkyl or (3-8C)cycloalkyl;
or R³ and R⁴ together with the nitrogen atom to which they are bonded are a nonaromatic (4-8)membered ring optionally containing another heteroatom, the ring optionally being substituted with (1-8C)alkyl or SO₂-(1-8C)alkyl;
**Q** is a spacer having a chain length of 10 to 70 atoms; and
**Z** is a negatively charged oligosaccharide residue comprising two to six monosaccharide units, the charge being compensated by positively charged counterions;
or a pharmaceutically acceptable salt thereof or a prodrug thereof as defined in claim 1 or solvate thereof,
for the manufacture of a medicament for the pulmonary delivery of said compound for the prevention or treatment of thrombosis or related disorders.

A preferred oligosaccharide conjugate for the use according to this invention is the compound of Formula (II) 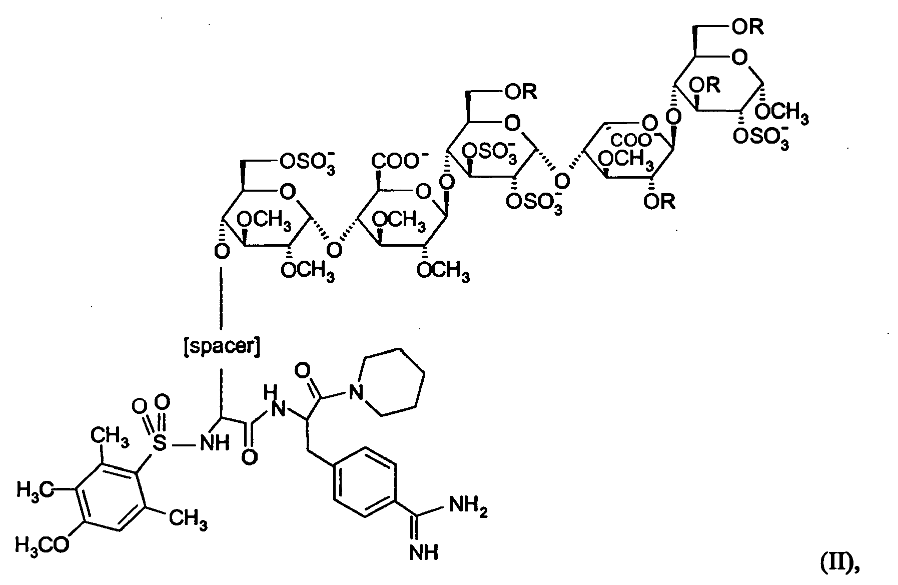
wherein R is independently SO₃⁻ or CH₃;
the spacer is a flexible spacer of a length of 13-25 atoms;
the charge of the pentasaccharide residue is compensated by positively charged counterions; and the total number of sulfate groups in the pentasaccharide residue is 4, 5 or 6;
or a pharmaceutically acceptable salt, a prodrug thereof as defined in claim 1 solvate thereof.

Particularly preferred is the compound of Formula (III) 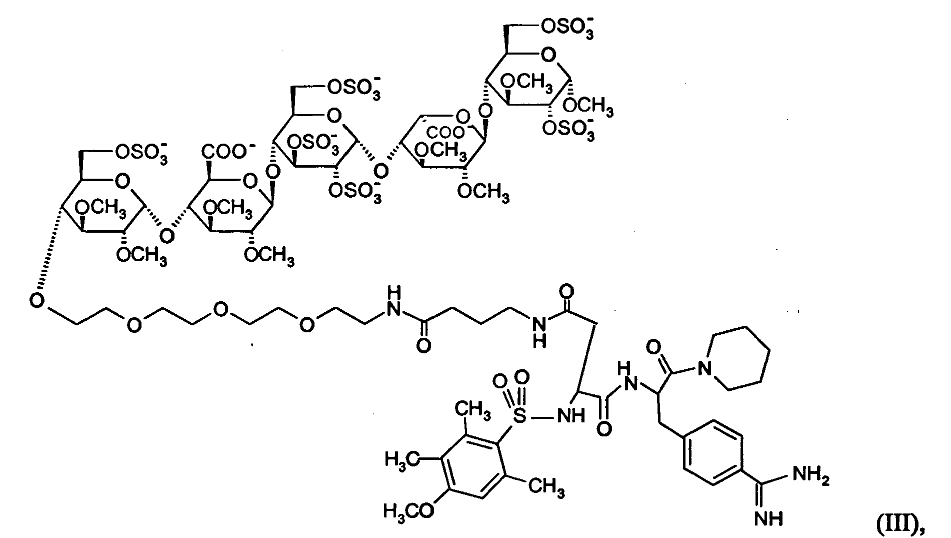 or a pharmaceutically acceptable salt, a prodrug thereof as defined in claim 1 or solvate thereof and specifically its octasodium salt, known by its code name Org 42675 (WO 01/42262; J. Thromb. Haem. 2003; 1: 1945-1954).

The use of the present invention comprises the use of the oligosaccharide conjugate either per *se,* without pharmaceutical auxiliaries, or a pharmaceutical composition thereof for pulmonary administration to a patient in need of treatment. The patient is a mammal and, usually, a human being.

The chemical structure of the spacer, as defined herein, is of minor or no importance for the antithrombotic activity of the compounds of the invention, it may however not be completely rigid. Highly flexible spacers are more preferred than others. Specific and highly suitable spacers are described in WO99/65934 and WO 01/42262.

The term (1-8C)alkyl means a branched or unbranched alkyl group having 1-8 carbon atoms, for example methyl, ethyl, propyl, isopropyl, butyl, sec-butyl, tert-butyl, hexyl and octyl. Methyl and ethyl are preferred alkyl groups.

The term (1-6C)alkoxy means an alkoxy group having 1-6 carbon atoms, the alkyl moiety having the meaning as previously defined. Methoxy is a preferred alkoxy group.

The term (3-8C)cycloalkyl means a cycloalkyl group having 3-8 carbon atoms, being cyclopropyl, cyclobutyl, cyclopentyl, cyclohexyl, cycloheptyl or cyclo-octyl. Cyclopentyl and cyclohexyl are preferred cycloalkyl groups.

A positively charged counterion means H⁺, Na⁺, K⁺, Ca²⁺, and the like. Preferably the oligosaccharide conjugate compounds are in the form of their sodium salt.

The term prodrug means a compound of the invention in which the amino group of the amidino-moiety is protected by hydroxy or a (1-6C)alkoxycarbonyl group.

Solvates according to the invention include hydrates.

The oligosaccharide conjugates of the invention may occur in the form of a free base, which may be isolated from the reaction mixture in the form of a pharmaceutically acceptable salt. The pharmaceutically acceptable salts may also be obtained by treating the free base of formula (I) with an organic or inorganic acid such as hydrogen chloride, hydrogen bromide, hydrogen iodide, sulfuric acid, phosphoric acid, acetic acid, propionic acid, glycolic acid, maleic acid, malonic acid, methanesulphonic acid, fumaric acid, succinic acid, tartaric acid, citric acid, benzoic acid, ascorbic acid and the like.

The oligosaccharide conjugate can be administered via the pulmonary route at several stages of the treatment according to the use of this invention.

For use according to the invention, the oligosaccharide conjugate may be administered in a dosage of 0.001-1.0 mg per kg body weight per day. More preferably, the conjugate is administered at doses of between 0.2 mg and 5 mg per patient per day.

The oligosaccharide conjugate may be used alone or may be presented as a pharmaceutical composition.

The term acceptable means being compatible with the other ingredients of the composition and not deleterious to the recipients thereof.

For administration according to the invention, the oligosaccharide conjugate may be presented alone, as dry particles, or as a pharmaceutical composition, either as dry particles further comprising pharmaceutically acceptable auxiliaries, or in a pharmaceutically acceptable liquid, optionally further comprising pharmaceutically acceptable auxiliaries. Preferably, the pharmaceutical composition is an isotonic solution of the oligosaccharide compound. The formulation is presented preferably in unit-dosages, in unit-dose or multi-dose containers, e.g. inhalation compositions for use in insufflators or inhalators in predetermined amounts.

Mixed with such pharmaceutically acceptable auxiliaries, e.g. as described in the standard reference, Gennaro et al., Remington's Pharmaceutical Sciences, (20th ed., Mack Publishing Company, see especially Part 5: Pharmaceutical Manufacturing, in particular also Chapter 50: Aerosols), and optionally by means of pharmaceutically acceptable liquids the oligosaccharide conjugate may be processed as an aerosol, or as a fluid composition, in the form of a solution, suspension, emulsion, or as a spray. Such composition may include a pulmonary delivery enhancer, e.g., a surfactant. Also aqueous suspensions, isotone saline solutions may be used, containing pharmaceutically acceptable dispersing agents and/or wetting agents, such as propylene glycol or butylene glycol.

A suitable aqueous formulation for use in this invention is a solution of the oligosaccharide conjugate in an isotonic phosphate-citrate buffer. Preferably, the pH is kept between 5 and 6. Alternative buffers may also be used with the same effect, such as - but not limited to - acetate and maleic acid, preferably in the pH range 5 to 6.

The pharmaceutical composition according to the invention may also be presented in the form of a veterinary composition, such compositions may be prepared by methods conventional in the art.

Also an embodiment of the present invention the use of the oligosaccharide conjugate according to the present invention, where a medicament comprising said compound is placed into a device which is suitable for administering pharmaceutical compositions via the pulmonary route. Particularly preferred is a device which is adapted for administering measured dosages.

Devices suitable for pulmonary delivery are well known in the art, e.g. inhalators, insufflators and ventilators for inhalation through the mouth or nasal passage, such as described in the standard reference, Gennaro et al., Remington's Pharmaceutical Sciences, (20th ed., Mack Publishing Company, see especially Part 5: Pharmaceutical Manufacturing, Chapter 50: Aerosols) or in the European Pharmacopeia 4.4 (04/2003:0671), J. Clin. Pharm. 2003, 56, 588-599; J. Clin. Pharm. 2003, 56, 600-612, etc.. Also pressurized containers or dispensers may be considered, using a suitable propellant or nebulizer.

The invention is further illustrated by the following examples. This should not be considered to be limiting in any way.

### EXAMPLE 1

### Pharmaceutical formulation of Org 42675

A suitable aqueous pharmaceutical formulation for Org 42675 is a solution in an isotonic phosphate-citrate buffer comprising the following excipients:
Disodium hydrogen phosphate dihydrate: 10 mM
Citric acid monohydrate: 3 mM
pH =6.

### EXAMPLE 2

### Intratracheal/ intrapulmonary administration of Org 42675

### Comparative study between intravenous administration and intratracheal administration of Org 42765

The test was carried out in male Wistar rats of 300 - 400 gr. The rats were anaesthetised by inhalation of a mixture of O₂/N₂O/isoflurane, after which the right jugular vein was cannulated.

Translumination of the neck enabled visibility of the vocal cords and an oral needle was inserted when the vocal cords were open after which 100 µl/300 gr body weight of a solution prepared from the pharmaceutical formulation of Example 1, diluted with an aqueous 0.9 % sodium chloride solution, containing 300 nmol of Org 42675 / ml was instilled at approximately 1 cm before the bifurcation of the trachea. The same solution and dose was also used to perform the i.v. study. After i.v. administration blood was sampled at 1, 6, 15, 30 and 60 minutes and after 2, 4 , 7 and 24 hours. After intra-tracheal administration blood was sampled at 0.5 -1 -2 - 4 -7 and 24 hours after administration. Blood was centrifuged and the plasma was siphoned off and stored at -20°C until, use. The concentration of Org 42675 was measured amidolytically with S2222 (Chromogenix, Chromogenics Ltd, Molndal, Sweden) by determination of the anti-Xa activity based on the method of Teien and Lie in the obtained plasma samples against a calibration curve which was made of the stock solution of 300 nmol/ml itself. (Teien AN, Lie M. Evaluation of an amidolytic heparin assay method increased sensitivity by adding purified antithrombin III. Thromb. Res. 1977, 10: 399-410). The concentration in the samples was expressed in nmol/ml and the kinetic parameters were calculated with the noncompartment model of WinNonlin (see Tables 1 and 2). After intratracheal administration the maximum concentration was already obtained at 0.5 hour after administration which was also the first time point of blood collection.

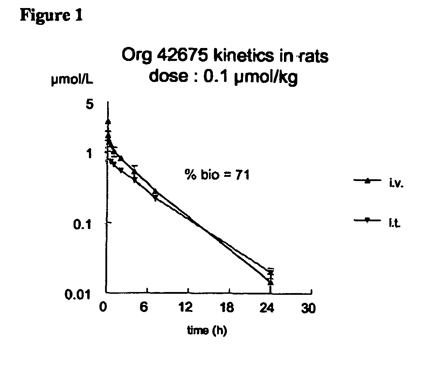

After i.v. administration an AUC_{inf} was obtained of 7.32 ± 1.03 (mean ± s.e.m. n=2) h.nmol/ml and after intratracheal administration the AUC_{inf} was 5.07 ± 0.24 (means s.e.m. n=3) h.nmol/ml. This means that the bioavailability after intratracheal administration is 71 ± 3 % (see Figure 1), where the bioavailability of i.v. administration is 100 %.

### Conclusion:

Org 42675 after intratracheal administration shows a pharmacokinetic performance which allows this route of administration to be used in home-medication and chronic anticoagulant therapy.

**Table 1**

| Org 42675 | 100 nmol/kg | | intravenous | |
|---|---|---|---|---|
| | rat1 | rat 2 | mean | s.e.m. |
| Cmax (nmol/ml) | 3.1815 | 2.6744 | 2.93 | |
| AUClast (h.nmol/ml) | 8.3527 | 6.2929 | 7.32 | 1.03 |
| AUCinf (h.nmol/ml) | 8.459 | 6.3462 | 7.40 | 1.06 |

**Table 2**

| Org 42675 | 100 nmol/kg | | intratracheal | | |
|---|---|---|---|---|---|
| | rat1 | rat2 | rat 3 | mean | ±s.e.m. |
| Tmax (h) | 0.5 | 0.5 | 0.5 | 0.5 | |
| Cmax (nmol/ml) | 0.64 | 0.72 | 0.81 | 0.77 | 0.05 |
| AUClast (h.nmol/ml) | 5.3968 | 5.205 | 4.6205 | 4.91 | 0.29 |
| AUCinf (h.nmol/ml) | 5.5283 | 5.3083 | 4.8327 | 5.07 | 0.24 |
| F (% bioavailabilty) * | 75.5 | 72.5 | 66.0 | 71.3 | 2.8 |

## Claims

1. Use of a compound of the formula (I) 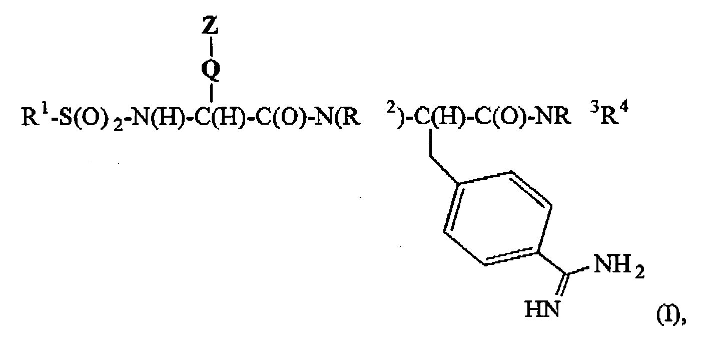
wherein R¹ is phenyl, naphthyl, 1,2,3,4-tetrahydronaphthyl, (iso)quinolinyl, tetrahydro(iso)quinolinyl, 3,4-dihydro-1H-isoquinolinyl, chromanyl or the camphor group, which groups may optionally be substituted with one or more substituents selected from (1-8C)alkyl or (1-8C)alkoxy;
R² and R³ are independently H or (1-8C)alkyl;
R⁴ is (1-8C)alkyl or (3-8C)cycloalkyl;
or R³ and R⁴ together with the nitrogen atom to which they are bonded are a nonaromatic (4-8)membered ring optionally containing another heteroatom, the ring optionally being substituted with (1-8C)alkyl or SO₂-(1-8C)alkyl;
Q is a spacer having a chain length of 10 to 70 atoms; and
Z is a negatively charged oligosaccharide residue comprising two to six monosaccharide units, the charge being compensated by positively charged counterions;
or a pharmaceutically acceptable salt thereof or a prodrug thereof wherein the amino group of the amidino- moiety is protected by hydroxy or a (1-b C) alkoxycarbonyl or solvate thereof,
for the manufacture of a medicament for the pulmonary delivery of said compound for the prevention or treatment of thrombosis or related disorders.

2. The use according to claim 1, wherein the compound is the compound of Formula (II) 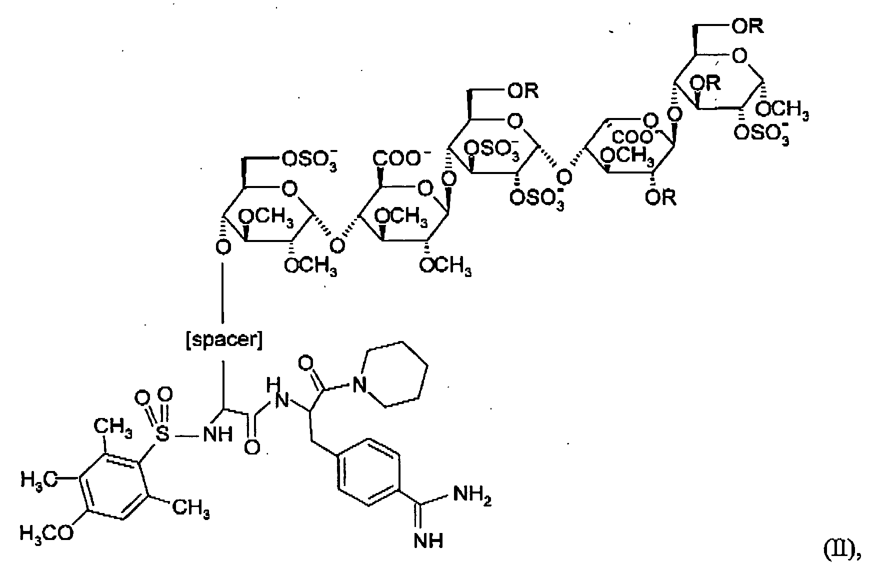
wherein R is independently SO₃⁻ or CH₃;
the spacer is a flexible spacer of a length of 13-25 atoms;
the charge of the pentasaccharide residue is compensated by positively charged counterions;
and the total number of sulfate groups in the pentasaccharide residue is 4, 5 or 6;
or a pharmaceutically acceptable salt, aprodrug thereof wherein the amino group of the amidino- moiety is protected by hydroxy or a (1-b C) alkoxycarbonyl or solvate thereof.

3. The use of claim 2, wherein the compound is the compound of Formula (III) 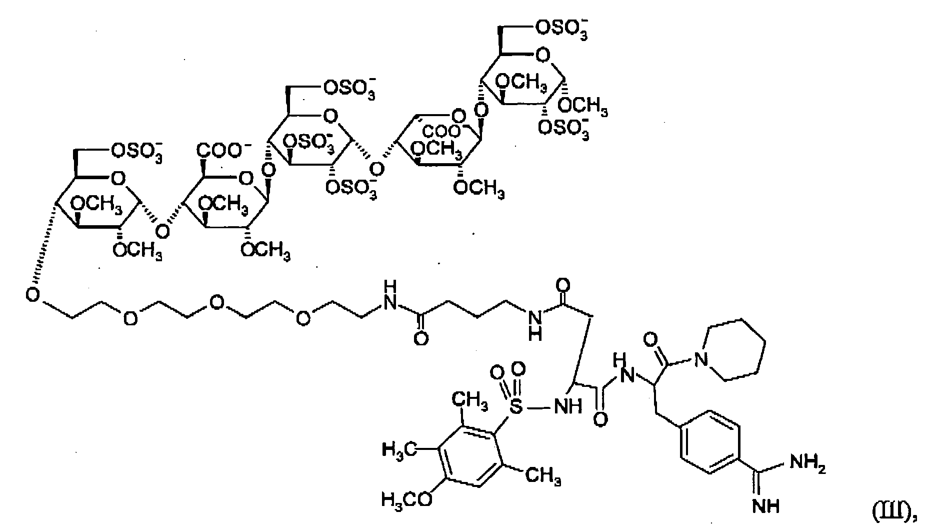 or a pharmaceutically acceptable salt, aprodrug thereof wherein the amino group of the amidino- moiety is protected by hydroxy or a (1-b C) alkoxycarbonyl or solvate thereof.

4. The use according to any one of claims 1-3, wherein the compound of Formula (III) is in the form of its octasodium salt.

5. The use according to any one of claims 1-4, wherein the medicament comprises the compound and a pharmaceutically acceptable liquid.

6. The use according to claim 5, wherein the medicament is an isotonic solution of the compound.

7. The use according to any one of claims 1-4, wherein the medicament comprises the compound as a dry particle.

8. The use according to any one of claims 5 - 7, wherein the medicament further comprises pharmaceutically acceptable auxiliaries.

9. The use according to any one of claims 1 - 8, wherein the medicament is provided in unit dosages.

10. The use according to any one of claims 1-9, wherein the medicament is placed into a device which is suitable for administering pharmaceutical compositions via the pulmonary route.

11. The use according to claim 10, wherein the device is adapted for adminstering measured dosages.

## Patentansprüche

1. Verwendung einer Verbindung der Formel (I) 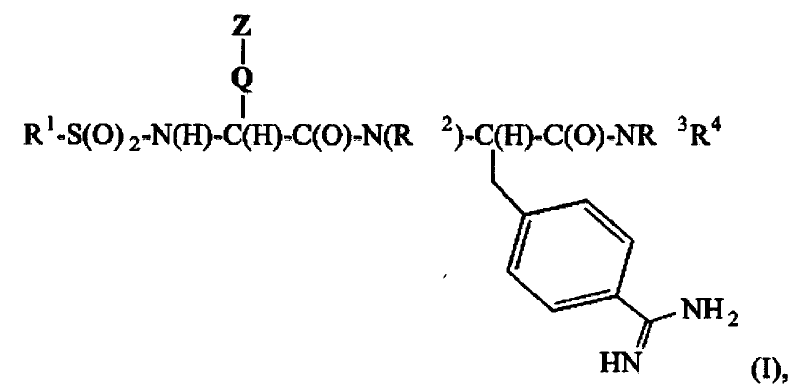
wobei R¹ Phenyl, Naphthyl, 1,2,3,4-Tetrahydronaphthyl, (iso)Chinolinyl, Tetrahydro(iso)chinolinyl, 3,4-Dihydro-1H-isochinolinyl, Chromanyl oder die Kampfer-Gruppe ist, welche Gruppen optional substituiert sein können mit einem oder mehreren Substituenten ausgewählt aus (1-8C)Alkyl oder (1-8C)Alkoxy;
R² und R³ unabhängig voneinander H oder (1-8C)Alkyl sind;
R⁴ (1-8C)Alkyl oder (3-8C)Cycloalkyl ist;
oder R³ und R⁴ zusammen mit dem Stickstoffatom, an dem sie gebunden sind, einen nicht-aromatischen (4-8)-elementigen Ring sind, optional ein anderes Heteroatom enthaltend, wobei der Ring optional mit (1-8C)Alkyl oder SO₂-(1-8C)Alkyl substituiert ist;
Q ein Abstandhalter mit einer Kettenlänge von 10 bis 70 Atomen ist; und
Z ein negativ geladenes Oligosaccharidresiduum ist, umfassend zwei bis sechs Monosaccharideinheiten, wobei die Ladung durch positiv geladene Gegenionen kompensiert wird;
oder ein pharmazeutisch verträgliches Salz davon oder ein Prodrug davon, wobei die Aminogruppe der Amidino-Moietät durch eine Hydroxy- oder eine (1-6C)Alkoxycarbonyl-Gruppe geschützt ist,
oder ein Solvat davon, für die Herstellung eines Medikamentes für die pulmonale Abgabe der besagten Verbindung für die Vorbeugung oder Behandlung von Thrombose oder diesbezüglichen Störungen.

2. Verwendung nach Anspruch 1, wobei die Verbindung die Verbindung der Formel (II) ist 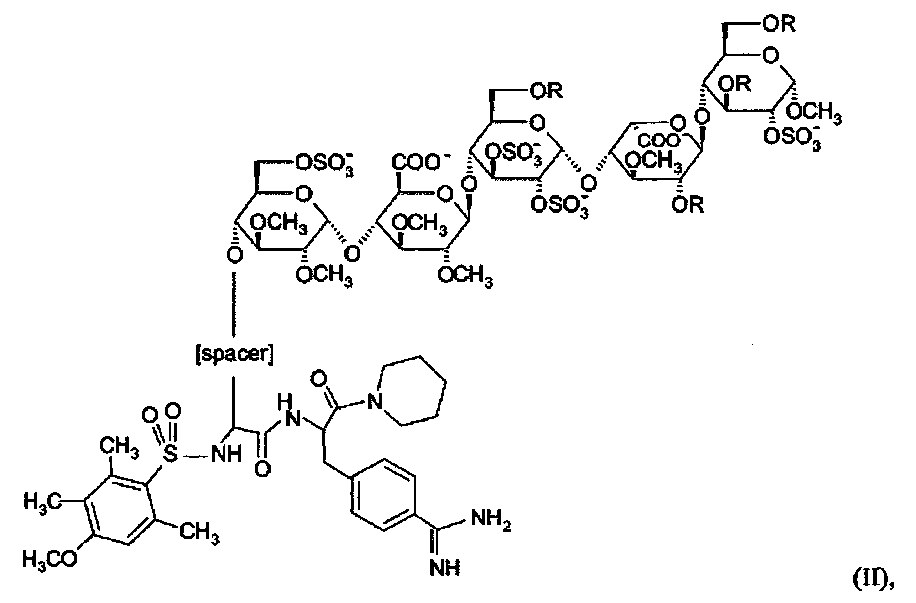
wobei R unabhängig SO₃⁻ oder CH₃ ist;
der Abstandhalter ein flexibler Abstandhalter einer Länge von 13-25 Atomen ist;
die Ladung des Pentasaccharidresiduums durch positiv geladene Gegenionen kompensiert ist;
und die Gesamtanzahl von Sulfatgruppen in dem Pentasaccharidresiduum 4, 5 oder 6 ist;
oder ein pharmazeutisch verträgliches Salz, ein Prodrug davon, wobei die Aminogruppe der Amidino-Moietät durch eine Hydroxy-oder eine (1-6C)Alkoxycarbonyl-Gruppe geschützt ist, oder ein Solvat davon.

3. Verwendung nach Anspruch 2, wobei die Verbindung die Verbindung der Formel (III) ist 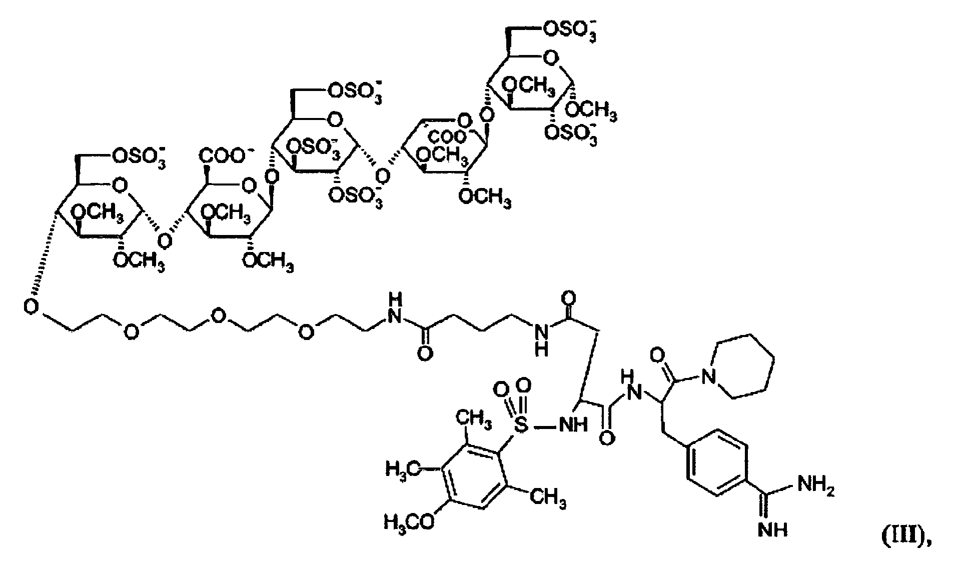 oder ein pharmazeutisch verträgliches Salz, ein Prodrug davon, wobei die Aminogruppe der Amidino-Moietät durch eine Hydroxy-oder eine (1-6C)Alkoxycarbonyl-Gruppe geschützt ist, oder ein Solvat davon.

4. Verwendung nach einem der Ansprüche 1 - 3, wobei die Verbindung der Formel (III) in Gestalt ihres Oktanatrium-Salz vorliegt.

5. Verwendung nach einem der Ansprüche 1 - 4, wobei das Medikament die Verbindung und eine pharmazeutisch verträgliche Flüssigkeit umfasst.

6. Verwendung nach Anspruch 5, wobei das Medikament eine isotonische Lösung der Verbindung ist.

7. Verwendung nach einem der Ansprüche 1 - 4, wobei das Medikament die Verbindung als ein trockenes Partikel umfasst.

8. Verwendung nach einem der Ansprüche 5 - 7, wobei das Medikament weiterhin pharmazeutisch verträgliche Hilfsstoffe umfasst.

9. Verwendung nach einem der Ansprüche 1 - 8, wobei das Medikament in Dosiseinheiten geliefert ist.

10. Verwendung nach einem der Ansprüche 1 - 9, wobei das Medikament in eine Vorrichtung anordbar ist, welche für das Verabreichen von pharmazeutischen Zusammensetzungen über den pulmonalen Weg geeignet ist.

11. Verwendung nach Anspruch 10, wobei die Vorrichtung für das Verabreichen von abgemessenen Dosen ausgestaltet ist.

## Revendications

1. Utilisation d'un composé de formule (I) 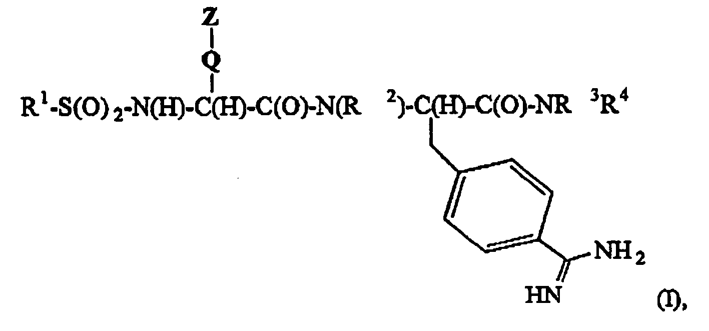
dans laquelle R¹ représente un groupe phényle, naphtyle, 1,2,3,4-tétrahydronaphthyle, (iso)quinoléinyle, tétrahydro(iso)quinoléinyle, 3,4-dihydro-1H-isoquinoléinyle, chromanyle ou un groupe camphre, lesquels groupes peuvent éventuellement être substitués par un ou plusieurs substituants choisis parmi un groupe alkyle en C₁ à C₈ ou alcoxyle en C₁ à C₈ ;
R² et R³ représentent chacun, indépendamment l'un de l'autre, H ou un groupe alkyle en C₁ à Cg ;
R⁴ représente un groupe alkyle en C₁ à C₈ ou cycloalkyle en C₃ à C₈ ;
ou R³ et R⁴ forment conjointement avec l'atome d'azote auquel ils sont liés un cycle non-aromatique de 4 à 8 éléments contenant éventuellement un autre hétéroatome, ce cycle étant éventuellement substitué par un groupe alkyle en C₁ à C₈ ou SO₂-(alkyle en C₁ à C₈);
Q représente un espaceur avec une longueur de chaîne de 10 à 70 atomes ; et
Z représente un résidu oligosaccharide chargé négativement comprenant deux à six motifs monosaccharide, la charge étant compensée par des contres-ions chargés positivement ;
ou un de ses sels acceptables sur le plan pharmaceutique ou un promédicament de celui-ci, dans lequel le groupe amino de la fraction amidino est protégé par un groupe hydroxyle ou (alcoxy en C₁ à C₆)-carbonyle ou un solvate de celui-ci,
à des fins de fabrication d'un médicament en vue d'une délivrance pulmonaire dudit composé pour la prévention ou le traitement de la thrombose ou des troubles liés.

2. Utilisation selon la revendication 1, dans laquelle le composé représente le composé de formule (II) 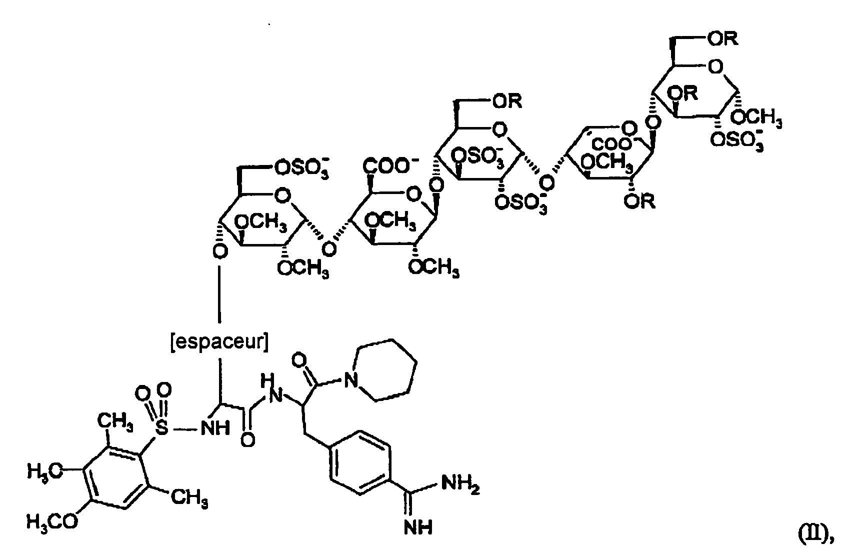
dans laquelle R représente indépendamment un groupe SO₃⁻ ou CH₃- ;
l'espaceur est un espaceur flexible d'une longueur de 13 à 25 atomes ;
la charge du résidu pentasaccharide est compensée par des contre-ions chargés positivement ;
et le nombre total de groupes sulfate dans le résidu pentasaccharide est de 4, 5 ou 6;
ou un sel acceptable sur le plan pharmaceutique de celui-ci, un promédicament de celui-ci, dans lequel le groupe amino de la fraction amidino est protégé par un groupe hydroxyle ou (alcoxy en C₁ à C₆)-carbonyle ou un solvate de celui-ci.

3. Utilisation selon la revendication 2, dans laquelle le composé représente le composé de formule (III) 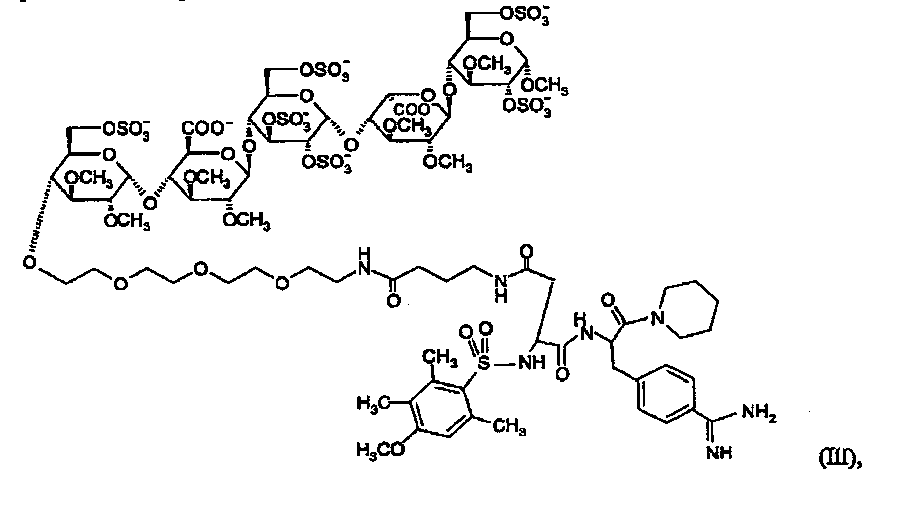 ou un sel acceptable sur le plan pharmaceutique de celui-ci, un promédicament de celui-ci, dans lequel le groupe amino de la fraction amidino est protégé par un groupe hydroxyle ou (alcoxy en C₁ à C₆)-carbonyle ou un solvate de celui-ci,

4. Utilisation selon l'une quelconque des revendications 1 à 3, dans laquelle le composé de formule (III) est sous la forme de son sel d'octasodium.

5. Utilisation selon l'une quelconque des revendications 1 à 4, dans laquelle le médicament comprend le composé et un liquide acceptable sur le plan pharmaceutique.

6. Utilisation selon la revendication 5, dans laquelle le composé est une solution isotonique du composé.

7. Utilisation selon l'une quelconque des revendications 1 à 4, dans laquelle le médicament comprend le composé sous la forme de particules sèches.

8. Utilisation selon l'une quelconque des revendications 5 à 7, dans laquelle le médicament comprend en outre des adjuvants acceptables sur le plan pharmaceutique.

9. Utilisation selon l'une quelconque des revendications 1 à 8, dans laquelle le médicament est conditionné en doses individuelles.

10. Utilisation selon l'une quelconque des revendications 1 à 9, dans laquelle le médicament est placé dans un dispositif adapté à l'administration de compositions pharmaceutiques par voie pulmonaire.

11. Utilisation selon la revendication 10, dans laquelle le dispositif est adapté à l'administration de doses mesurées.
